# EUROPEAN PATENT SPECIFICATION

(11) **EP 4 223 381 B1**
(45) Date of publication and mention of the grant of the patent: **02.04.2025**
(21) Application number: 22154718.5
(22) Date of filing: 02.02.2022
(51) Int. Cl.: B01D 15/08, B01D 15/16, B01D 15/22, A61K 35/16, C07K 1/16, C07K 14/745, G01N 33/50

(54) **METHOD OF PURIFYING OR REMOVING A TARGET COMPONENT FROM FULL PLASMA**
VERFAHREN ZUR REINIGUNG ODER ENTFERNUNG EINER ZIELKOMPONENTE AUS VOLLPLASMA
PROCÉDÉ DE PURIFICATION OU D'ÉLIMINATION D'UN COMPOSANT CIBLE À PARTIR DE PLASMA COMPLET

(43) Date of publication of application: 09.08.2023
(73) Proprietor: Sartorius BIA Separations d.o.o., 5270 Ajdovscina (SI)
(72) Inventor: Josic, Djuro, 1020 Wien (AT); Begic, Marija, 5250 Nova Gorcia (SI); Simovic Medica, Jasmina, 52100 Pula (HR)
(74) Representative: Novagraaf International SA

(56) References cited:
- EP-A1- 3 087 991
- WO-A1-2014/083510
- WO-A1-2021/084050
- WO-A2-2013/124474
- PETRIC ET AL: "Anion-exchange chromatography using short monolithic columns as a complementary technique for human serum albumin depletion prior to human plasma proteome analysis", JOURNAL OF PHARMACEUTICAL AND BIOMEDICAL ANALYSIS, ELSEVIER B.V, AMSTERDAM, NL, vol. 43, no. 1, 8 December 2006 (2006-12-08), pages 243 - 249, XP005798558, ISSN: 0731-7085, DOI: 10.1016/J.JPBA.2006.06.019
- IVANCIC-JELECKI J ET AL: "Isolation of cell-free DNA from plasma by chromatography on short monolithic columns and quantification of non-apoptotic fragments by real-time polymerase chain reaction", JOURNAL OF CHROMATOGRAPHY A, ELSEVIER, AMSTERDAM, NL, vol. 1216, no. 13, 27 March 2009 (2009-03-27), pages 2717 - 2724, XP026006269, ISSN: 0021-9673, [retrieved on 20081030], DOI: 10.1016/J.CHROMA.2008.10.087
- QUANZHOU LUO ET AL: "High-performance affinity chromatography with immobilization of protein A and L-histidine on molded monolith", BIOTECHNOLOGY AND BIOENGINEERING, JOHN WILEY, HOBOKEN, USA, vol. 80, no. 5, 26 September 2002 (2002-09-26), pages 481 - 489, XP071038080, ISSN: 0006-3592, DOI: 10.1002/BIT.10391

## Description

The present invention relates to a method of purifying a target component from a full plasma sample. The present invention further relates to a method of removing one or more target components from a full plasma sample.

Petric et al. describe anion-exchange chromatography using short monolithic columns as a complementary technique for human serum albumin depletion prior to human plasma proteome analysis in Journal of Pharmaceutical and Biomedical Analysis, vol. 43, pages 243 to 249. WO 2014/083510 A1 describes monolith-based pseudo-bioaffinity purification methods for factor viii and applications thereof. EP 3 087 991 A1 describes the composition of inter-alpha inhibitor proteins from human plasma for therapeutic use. Ivancic-Jelecki et al. describe the isolation of cell-free DNA from plasma by chromatography on short monolithic columns and quantification of non-apoptotic fragments by real-time polymerase chain reaction in Journal of Chromatography A, vol. 1216, pages 2717 to 2724. WO 2013/124474 A2 describes the chromatographic isolation of cells and other complex biological materials. Quanzhou Luo et al. describe high-performance affinity chromatography with immobilization of protein A and L-histidine on molded monolith in Biotechnology and Bioengineering, vol. 80, pages 481 to 489. WO 2021/084050 A1 describes cell selection and/or stimulation devices and methods of use.

Up to now, it has been extremely difficult to load thawed undiluted pool of fresh frozen plasma or a single patient's plasma directly to a chromatographic unit or other purification devices. The main challenges in processing and/or purification of full undiluted human plasma are its viscosity, fragility, and precipitation of plasma components caused by the activation of the coagulation cascade and proteolytical enzymes. Monolithic supports, also including membrane-based materials, have been recently used for processing cryopoor plasma and/or other high-value biopolymers that are secreted by cultured cells [2,3]. Usually, before plasma processing, sample dilution is necessary. The reason for dilution is frequent plugging of chromatographic units or low yield of target molecules. Plugging occurs when bulk chromatographic supports, as well as monoliths and/or membrane-based supports, are used for plasma processing. Additionally, plasma processing is routinely performed at low temperatures in order to prevent the degradation of sensitive molecules or nanoparticles of interest [4,5]. Almost all current plasma fractionation technologies still rely on two backbone processes that are based on cryoprecipitation [6,7] followed by cold ethanol precipitation associated with shifts in pH, temperature, and osmolality resulting in selective precipitations of proteins. In early times, before precipitation of cryoglobulins was introduced, the most abundant plasma proteins albumin and intravenous immunoglobulin (IVIG) were produced [8].

In the last thirty years, separation processes based on chromatography were combined with precipitation. This combination enabled the isolation of low abundance proteins from cryoprecipitate, cryopoor plasma, and from different Cohn fractions [6,9].

For example, reference [10] discloses a monolith-based pseudo-affinity purification method for isolation of human blood plasma-derived and/or recombinant clotting factor VIII (FVIII). Hydrophilic polymethacrylate-based monoliths are used for fast chromatographic separation of this plasma protein without significant loss of separation efficiency. However, a significant difference of this process compared to the present invention is that the starting material is either cell-culture supernatant and/or diluted human plasma. In both cases an extensive sample preparation by dilution and adjustment of pH is necessary. This preparation requires additional steps and increases both process time and cost [2].

Direct application of complete undiluted human plasma to chromatographic units reduces both process cost and time. It also enables further continuous processing by a combination of different chromatographic methods to isolate other biologically active proteins. Monoliths and membranes can also be stacked to isolate different target components in one chromatographic run [2,12]. This new strategy enables a faster process, lowers the costs as well as the risk of degradation, modification, and/or activation of plasma components of interest. The final result is a higher yield and better quality and safety of the product.

Direct (online) processing of patient blood and/or plasma for removal of low molecular toxic components that accumulate in these body fluids in the case of insufficient kidney function is a routine treatment known as hemodialysis [12,13]. However, removal or isolation of macromolecules and nanoparticles directly from patient blood is a more difficult process, and the presented chromatographic method is a large step in this direction.

Plasmapheresis is the removal, treatment, and return or exchange of blood or components thereof from and to the blood circulation [14]. Autologous plasmapheresis is defined as the temporal removal of blood plasma followed by the extracorporeal treatment of this body fluid and returning it to the same person as a therapy. In the case of plasma exchange, patient blood plasma is removed and exchanged with donated plasma or other donated blood products. Plasmapheresis of the autologous and exchange types is used to treat a variety of disorders, including those of the immune system and/or neurological disorders [14-17].

By using the herein described methods, patients' plasma that is separated by plasmapheresis can be forced through large-pore chromatographic units with immobilized affinity ligands that can bind target macromolecule, like different pathological antibodies, and purified plasma can be returned to the patient.

The herein described methods utilize a continuous chromatographic material such as a synthetic monolith or membrane-based support for the processing of full, undiluted human plasma. By use of such chromatographic material the inventors surprisingly found that the herein described methods are suitable for the processing/purification of full undiluted human plasma without resulting in a clogging of the chromatographic material and/or the activation of plasma components resulting in the activation of signaling pathways including the coagulation cascade and complement system.

Based on the above, an object of the invention is to provide a method of purifying a target component from a full plasma sample without the need for subjecting the plasma to any preparative steps, such as dilution and/or cryoprecipitation.

According to the present invention, the above object is achieved by providing a method of purifying a target component from a full plasma sample comprising the steps of:
- Applying full plasma to a continuous chromatographic material, the continuous chromatographic material being a compact monolithic material or a membrane adsorber and comprising continuous channels having a diameter of 4 to 15 µm, the chromatographic material comprising a chromatographic selection element for the target component;
- Performing chromatography at a temperature of 30 to 39 °C; and
- Recovering the target component from the continuous chromatographic material,
wherein full plasma is plasma that has not been subjected to dilution and/or cryoprecipitation.

According to this method, undiluted plasma is applied to a continuous chromatographic material. The component to be purified is bound to the chromatographic material, and in the next step, it is recovered by elution. This method can also be used to recover several different components from undiluted plasma by using different chromatographic approaches such as ion exchange chromatography, affinity chromatography (e.g. protein A), hydrophobic interaction chromatography, reverse phase chromatography and/or different elution methods. Accordingly, the method is suitable for purifying and separating target molecules from undiluted plasma that can be used in therapeutic applications.

A further object of the invention is to provide a method of removing one or more target components from a full plasma sample without having to subject the plasma to any preparative steps, such as dilution and/or cryoprecipitation.

According to the present invention, the above object is achieved by providing a method of removing one or more target components from a full plasma sample comprising the steps of:
- Applying full plasma to a continuous chromatographic material, the continuous chromatographic material being a compact monolithic material or a membrane adsorber and comprising continuous channels with a diameter of 4 to 15 µm, the chromatographic material comprising a chromatographic selection element for the one or more target components;
- Performing chromatography at a temperature of 30 to 39 °C; and
- Recovering the plasma after removal of the one or more target components, wherein full plasma is plasma that has not been subjected to dilution and/or cryoprecipitation.

This method can be used for therapeutic applications similar to the apheresis in which the removal of harmful components (e.g. autoimmune antibodies, allergens and the like) from the plasma is a critical step before the plasma is recirculated back to the patient.

The present invention is directed to the chromatographic separation and/or purification of full plasma. Suitable starting materials are fresh frozen plasma and patient plasma after temporary removal of cells by autologous plasmapheresis. In a preferred embodiment, the plasma is human blood plasma.

The target components to be either purified or removed can be selected from the group consisting of proteins, such as antibodies and allergens, nucleic acids, viruses, macromolecular toxins, and nanoparticles. Nanoparticles are, for example, exosomes and other microvesicles. Suitable proteins for fast isolation and therapeutic use are clotting factors and/or clotting inhibitors as well as antibodies. One specific target component is the clotting factor VIII.

According to the invention, a continuous chromatographic material is used. The continuous chromatographic material has channels within the material. The continuous chromatographic material is a compact monolithic material or a membrane adsorber and has continuous channels with a diameter of 4 to 15 µm. In a preferred embodiment, the continuous channels have a diameter in the range of 4 to 10 µm, more preferably 5 to 7 µm. The diameter of the channels represents the average diameter as determined by mercury porosimety.

Monoliths are generally regarded as an alternative to particle-based stationary phases in chromatography. Monoliths are made of a single molded structure characterized by a high inlet surface area. Due to the absence of diffusive pores, mass transfer takes place exclusively by convective forces.

One of the main advantages of continuous chromatographic materials, in particular CIM monoliths and membranes, is their consistency. There is a significant difference between continuous and bulk chromatographic materials. In monolithic and membrane-based materials interaction between ligands and ligates (components of the sample that is of interest and shall be isolated, and that binds to the ligand) occurs mainly in the channels, on the surface of continuous material. In "conventional", particle-based, bulk material, this interaction occurs in the pores inside the material and it is limited by diffusion. It also means that the surface inside pores of the bulk, particle-based material is critical for separation performance of the bulk material. On the other hand, an increase in surface area also increases the possibility for non-specific interactions. These interactions are responsible for non-specific bindings (that can be irreversible), but also for activation and aggregation of plasma components.

Additionally, the physical interaction occurs in the pores of the bulk material. Driving force here is diffusion and the physical interactions are therefore limited by the pore dimensions, surface area, and by flow rate. In monoliths and membranes, the interaction occurs in the channels of the material rather than in pores, and the driving force here is not diffusion, but convection (CIM - "Convective Interaction Media").

Pores may have a random orientation and comprise dead-ends, which increased the surface area without benefiting the throughput of a column. In contrast, the channels of a continuous chromatographic material continue from the beginning to the end of the column. In general, a larger diameter of the channels results in a lower surface area of the chromatographic material. This means that the surface of the continuous chromatographic material is a couple orders of magnitude lower, and that the level and danger of non-specific interactions and activations are likewise orders of magnitude lower [18].

In a preferred embodiment, the surface of the channels is hydrophilic and may comprise specific ligands immobilized on the surface. Due to the tailored dimension of the channels of the continuous chromatographic material the hydrophobicity in the channels is reduced compared to a bulk or micro/mesoporous material. Additionally, the limiting factor of pore diffusion is eliminated. Pore diffusion is mainly responsible for slow kinetics and, indirectly, for non-specific bindings and building of aggregates. By suitable tailoring of channels, destruction of fragile macromolecules and nanoparticles is significantly reduced.

In a further preferred embodiment the continuous chromatographic material is inert in nature thereby preventing direct interaction and activation of plasma components. Exemplary chromatographic materials which have an inert and/or hydrophilic character are made by a radical copolymerization of glycidyl methacrylate and ethylene dimethacrylate in the presence of pore-producing solvents following the method of Švec et al. [19,20].

According to the present invention, the continuous chromatographic material comprises a chromatographic selection element. This selection element is capable of selective binding to the target component to be purified or removed from the plasma.

Hydrophilic and inert character of the continuous chromatographic material may be further improved by applying specific surface chemistry. Suitable chemistry comprises introduction of polyethylenglycol (for a neutral and inert surface) and different ionexchange groups (ion-exchanger), hydrophobic molecules, and small and/or large molecules like enzymes, inhibitors, antibodies, allergens, polynucleotides and nucleic acids and nanoparticles for affinity and pseudoaffinity chromatography [21].

In a preferred embodiment, ligands can be immobilized on the surface of the chromatographic material and act as the chromatographic selection element. Depending on the type of target component to be bound by the column, suitable chromatographic ligands include anion exchange ligands (strong or weak), cation exchange ligands (strong or weak), hydrophobic-interaction ligands, low-molecular and/or macromolecular affinity ligands (such as protein A), oligonucleotides, aptamers, immobilized nanoparticles, and combinations thereof.

After application of the full plasma to the chromatographic material, chromatography is performed at a temperature of 30 to 39°C. This prevents denaturation, aggregation, and precipitation of macromolecular plasma components and nanoparticles and also reduces the viscosity of the plasma. The chromatography is preferably performed at a temperature between 35 and 37 °C. The use of a physiological temperature range during plasma processing is one key difference compared to commonly known approaches which are performed at low temperatures such as 4°C. At such low temperature full, undiluted plasma becomes highly viscous making processing without prior dilution impossible.

In a preferred embodiment, the chromatography is performed at a flow rate of 0.2 to 2 column volumes per minute (CV/min). That is, for example, for a 8ml column the flow rate lies between 1.6 and 16 mL/min. Such a flow rate represents a good balance between the time taken for chromatography and the resulting column pressure due to the consistency of the plasma. In addition, with such a flow rate, stability of plasma components, such as macromolecules and nanoparticles, can be ensured (see Refs. [1-3] and [18,20,21].

In the final step of the methods according to the present invention, the plasma after removal of the target component is recovered and the column comprising the target component may be discarded. Alternatively, the plasma may be discarded or used for isolation of further components, and the target component is eluted from the column to be recovered.

A typical recovery method is decreasing the binding forces of the target component to the chromatographic material. This can be achieved by using high salt concentrations, pH change of elution buffers, and by components with higher affinity to the chromatographic support.

### Brief description of the figures

- Figure 1: Chromatogram of Example 1: Loading of 150 ml of human plasma through a 1 ml CIMmultus OH column having a pore size of 6 µm (full line- 280nm in mAU; dashed line- conductivity in mS/cm).
- Figure 2: SDS-PAGE analysis of start sample (undiluted human plasma), flow-through fractions after 25 and 50ml of plasma passed through the column (FT25 and FT50), and eluates E1 and E2 of Example 1.
- Figure 3: Chromatogram of Example 2: Loading of 150 ml of human plasma through a 1 ml CIMmultus QA column having a pore size of 6 µm (full line- 280nm in mAU; dashed line- conductivity in mS/cm).
- Figure 4: SDS-PAGE analysis of plasma and chromatography fractions from QA column; analysed are flow-through fractions after 25, 75 and 100 ml of plasma passed through the column (FT25, FT75, and FT100) and elutions E1, E2 and E3 of Example 2.
- Figure 5: Chromatogram of Example 3: Chromatography on protein G column: Isolation of IgG from undiluted human plasma (full line- 280nm in mAU; dashed line- conductivity in mS/cm).
- Figure 6: SDS-PAGE analysis of human plasma, flow-through fractions FT and eluate; heavy chains of approximately 50 kDa and light chains of approximately 25 kDa are visible in protein G eluate lane (Example 3).

In summary, the methods of the present invention may be employed for the purpose of purifying a target component from a full plasma sample and for removing one or more target components from a full plasma sample without the need for pre-processing of the plasma, such as dilution and/or cryoprecipitation and without the risk of clogging the continuous chromatographic material due to plasma viscosity and/or activation of the coagulation cascade.

The present invention will be further illustrated in the following examples without being limited thereto.

### Examples

### Materials used in Examples 1 to 3

- Fresh-frozen human plasma (FFP), 260 mL
- CIMmultus OH, 1mL column (6 µm pore diameter), BIASeparations, Ajdovščina, Slovenia
- CIMmultus QA, 8mL strong anion-exchange column (6 µm pore diameter), BIASeparations, Ajdovščina, Slovenia
- CIMmultus r-Protein G, 1mL column (1.3 µm and 6 µm pore diameter), BIASeparations, Ajdovščina, Slovenia
- Coagulation factor VIII deficient plasma, Siemens Healthcare GmbH, Erlangen, Germany, Lot 547664A
- Dade Actin FS Activated PTT Reagent, Siemens Healthcare GmbH, Erlangen, Germany, Lot 538564.

### Example 1: Flow of human plasma through a hydroxyl modified column

Fresh frozen plasma (FFP) was thawed, centrifuged at 10,000g for 20 minutes at 36°C and the lipid layer was removed. 150 ml of human plasma was pumped through a 1 ml CIMmultus OH column having a pore size of 6 µm (available from BIA Separations, Slovenia) at a flow rate of 1.0 to 5.0 mL/min (0.2-1.0 CV/min, see Ref. [20]). The pressure was between 0.10 and 0.20 mPa, the temperature was set to 36 °C, and it was kept at this value during the whole process. An Äkta Explorer chromatographic system (Cytiva, Freiburg/Breisgau, Germany) was used.

The used continuous chromatographic unit has hydrophilic, inert OH-groups immobilized on the inner surface of a monolith having large channels with an average diameter of the channels of the monolith of 6 µm. This chromatographic material has an inert surface and a flow through of undiluted human plasma is possible without activating the coagulation cascade. In order to demonstrate this aspect, human plasma was pumped through the chromatographic unit without binding of plasma components, plugging, aggregation, activation, and/or precipitation of proteins and other macromolecules and nanoparticles that are contained, i.e. solved and/or emulsified, in this biological fluid.

Before application, the CIMmultus column was washed and equilibrated with a 50mM phosphate buffer pH 7.2(Buffer A), containing 155 mM NaCl with a conductivity of 15.9 mS/cm. After adjusting the temperature to 36°C and the flow rate to 3mL/min, 150 ml of human plasma passed the column without plugging (no increase in backpressure was registered). Samples were taken after thawing (FFP-1), prior to centrifugation (FFP-2), after centrifugation (FFP-3), and after pumping the following volumes of FFP through the column: 25 ml, 50 ml, 100 ml, and 150 ml. Non-specifically bound proteins were eluted in two steps with elution buffers, containing 0.25M NaCl in 50mM phosphate buffer, pH6.95 and a conductivity of 25.8 mS/cm (Buffer B) and 0.5M NaCl in 50mM phosphate buffer, pH7.2 and a conductivity of 25.8 mS/cm (Buffer C).

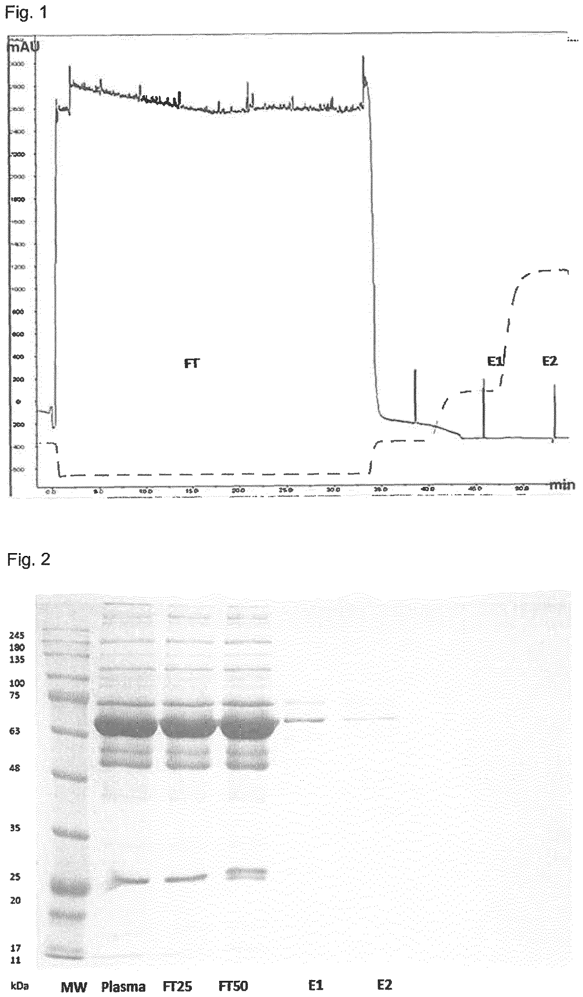

Figure 1 shows the obtained chromatogram, and Table 1 lists values for the measured coagulation status of the plasma and possible complement activation before and after sample preparation and further processing of the human plasma. The tests for determination of clotting factor VIII, partial-thromboplastine time (PTT) and activated partial thromboplastin time (APTT) were performed on BCS XP, Siemens (Siemens Healthineers, Munich, Germany), in the General Hospital Pula, Department of Transfusion Medicine.

Principle of the coagulation measurement method: Factor VIII Deficient Plasma, an in-vitro diagnostic reagent for the determination of the activity of coagulation factor VIII, was used and the clotting activity of added sample was determined according to manufacturer's protocol. For details see Ref. [21]. For determination of PTT and APTT, Dade Actin FS Activated PTT Reagent (Siemens) was used. Factors for the intrinsic coagulation system are activated by incubating the plasma with optimal amount of phospholipids and a surface activator. The addition of calcium ions triggers the coagulation process, and the clotting time is measured. For details see Refs. [22,23]. The tests for Complement C1 (esterase inhibitor), C3 and C4 were performed on a Cobas C503 analytical unit (Roche Diagnostics, Rotkreuz, Switzerland) in the Department of Clinical Chemistry, General Hospital Pula. The Berichrom C1-Inhibitor Kit (Sigma Aldrich, Merck,KGaA, Darmstadt, Germany), a human C1 esterase-based assay, determines the presence of C1 inhibitors in patient samples [24]. This chromogenic activity reagent is used for the diagnosis of angioneurotic edema and for monitoring substitution or steroid therapy in angioneurotic edema. Complement C3 determination was performad by use immunoturbidimetric assay (Abcam, Cambridge, UK) where the human C3c forms a precipitate with a specific antiserum which is determined turbidimetrically. Complement C4 activity was determinated by use of an immunoturbidimetric procedure that measures increasing sample turbidity caused by the formation of insoluble immune complexes when antibody to C4 (Abcam) is added to the sample [24].

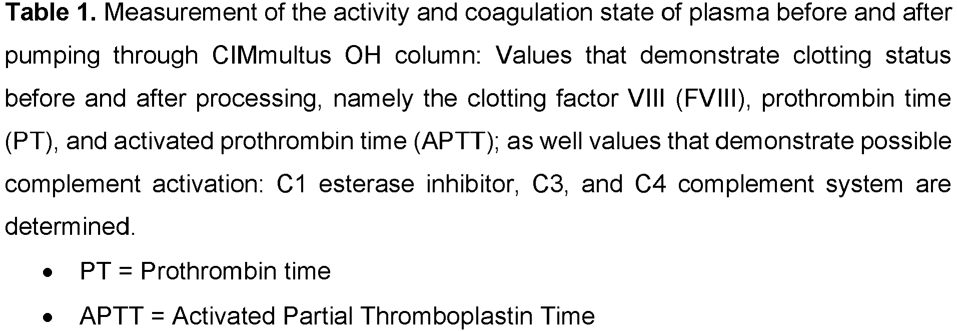

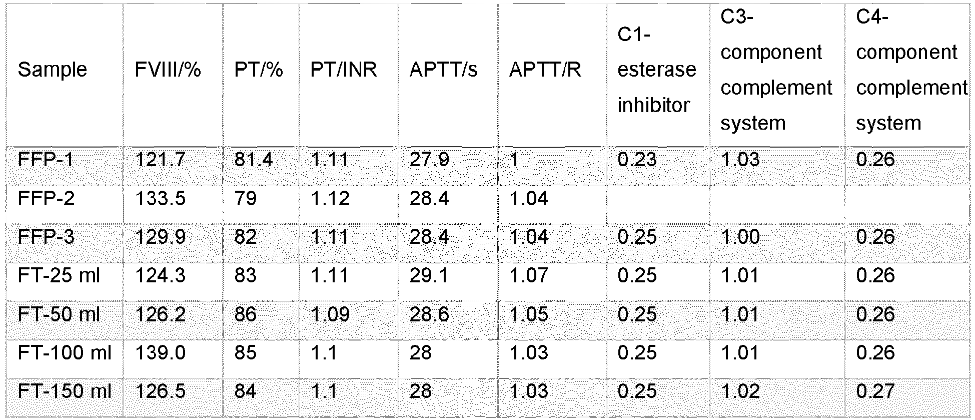

As shown in Table 1, pumping through the OH column did not influence the coagulation state of the plasma (FVIII activity, PT, and APTT), nor caused the activation of complement components C1, C3, and C4.

In summary, based on the above described experiments and the obtained experimental data it was concluded that after optimization of temperature [36°C] and flow rate [3mL/min], 150 mL of human plasma passed the column without plugging, and surprisingly, no change of activity of clotting FVIII and PTT and APTT, nor activation of complement components occurred. The unspecific binding and loss of plasma components were practically zero, as shown by the absence of any substantial bands in the elution fractions E1 and E2 in the SDS-PAGE in Figure 2.

Thus, it was further concluded that the OH-derived CIMmultus column can be used for direct processing of undiluted human plasma without the activation of the clotting cascade and the complement. If necessary, processed human plasma can be returned to the patient's blood. The described experiment therefore provides proof of concept that a monolithic material having a surface immobilized with inert hydrophilic ligands can be used for the processing and/or purification of fresh undiluted human plasma which provides a strong tool in a variety of therapeutic applications.

### Example 2: Processing of undiluted plasma on the strong ion exchanger

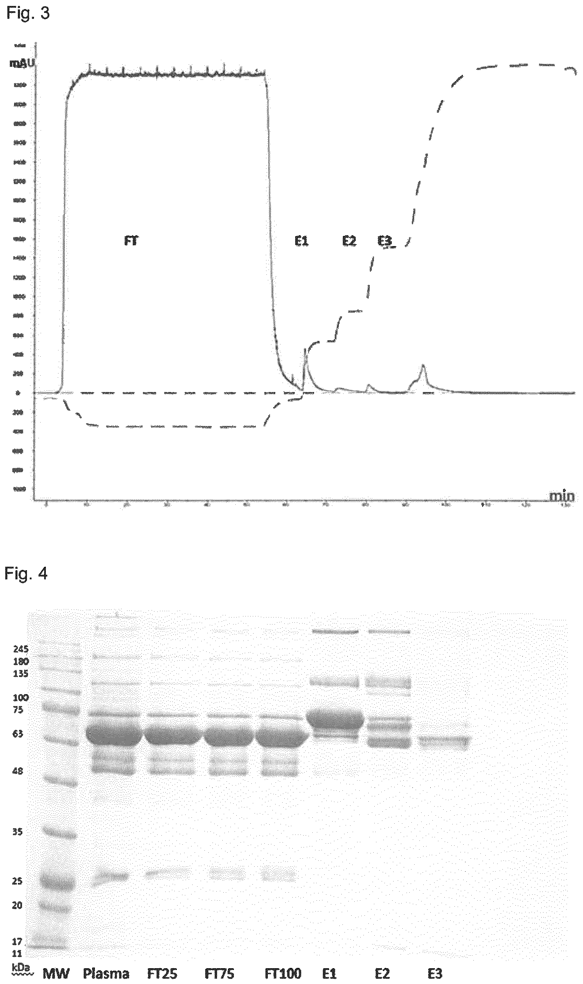

Fresh frozen plasma was thawed, centrifuged and the lipid layer was removed. 150 ml of the sample was pumped through an 8 ml CIMmultus QA column having a pore size of 6 µm (available from BIA Separations, Slovenia) at a flow rate of 3.0 ml/min. FVIII and other proteins with affinity to the columns were bound and could be eluted (see Figure 3). Clotting factor VIII was chosen as a model protein for this experiment. Equilibration and wash buffer for CIMmultus QA column was 20 mM phosphate buffer, pH 7.0 containing 155 mM NaCl, pH 7.0 with a conductivity of 17.6 mS/cm. Stepwise elution was performed with 280 mM NaCl in 20 mM phosphate buffer, pH 7.0 and 27.9 mS/cm (Buffer 1), 360 mM NaCl in 20 mM phosphate buffer, pH 7.0 and 34.7 mS/cm (Buffer 2) and 500 mM NaCl in 20 mM phosphate buffer, pH 7.0 and 47.0 mS/cm. The chromatogram is shown in Figure 3. Fractions were collected and analyzed for protein content and clotting factor VIII activity (see Table 2), and protein pattern was analyzed by SDS-PAGE (see Figure 4).

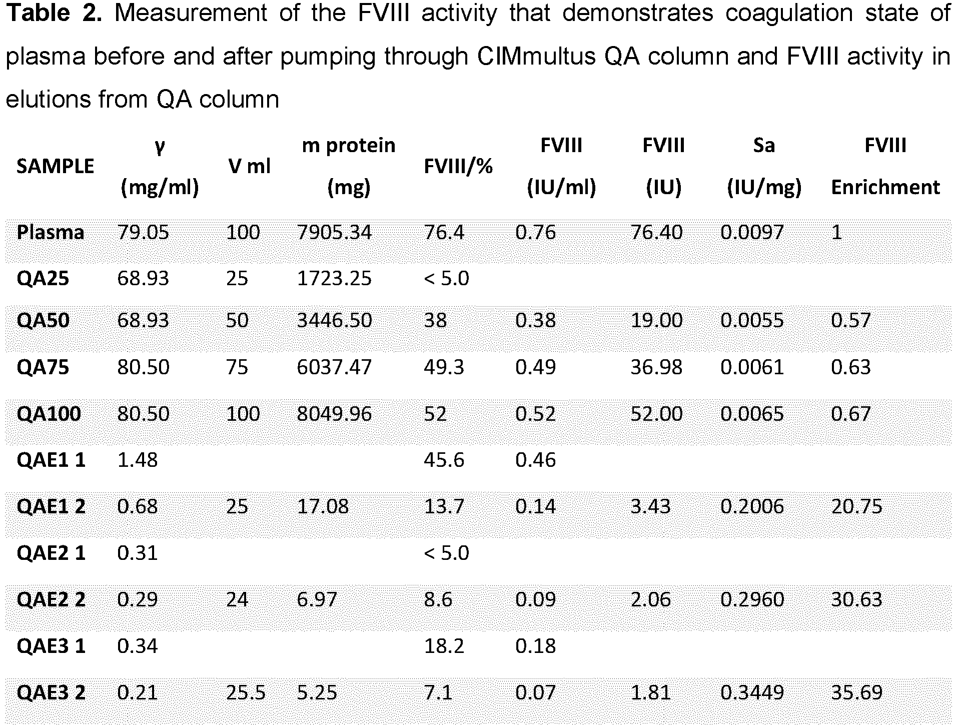

In Table 2, γ represents the protein concentration in the sample. Samples ending in -1 were taken at the beginning of the elution of a fraction while samples ending in -2 were taken at the end of an elution.

As shown in Table 2, after loading of 25mL plasma, factor VIII was bound to the column and could not be detected in the flow-through fraction (see fraction QA25 in Table 2). Upon overloading, FVIII is displaced from the column by other proteins with higher affinity to this anion-exchanger and it can be detected in further fractions. Bound proteins can be eluted. As can be seen in the SDS-PAGE in Figure 4 plasma proteins are stable throughout the experiment and not affected by passing through the column, as suggested by the unchanged profile of initial plasma and flow-through fractions.

Conclusion: From experiments 1 and 2 it may be derived that binding of plasma components followed by elution is possible without any plugging or precipitation during the processing of this biological fluid. The described experiments therefore provide proof that the herein described methods are suitable to process full undiluted plasma for therapeutic applications and/or to purify/remove (harmful) target substances from full undiluted human plasma.

### Example 3: Processing of undiluted human plasma with protein G affinity column

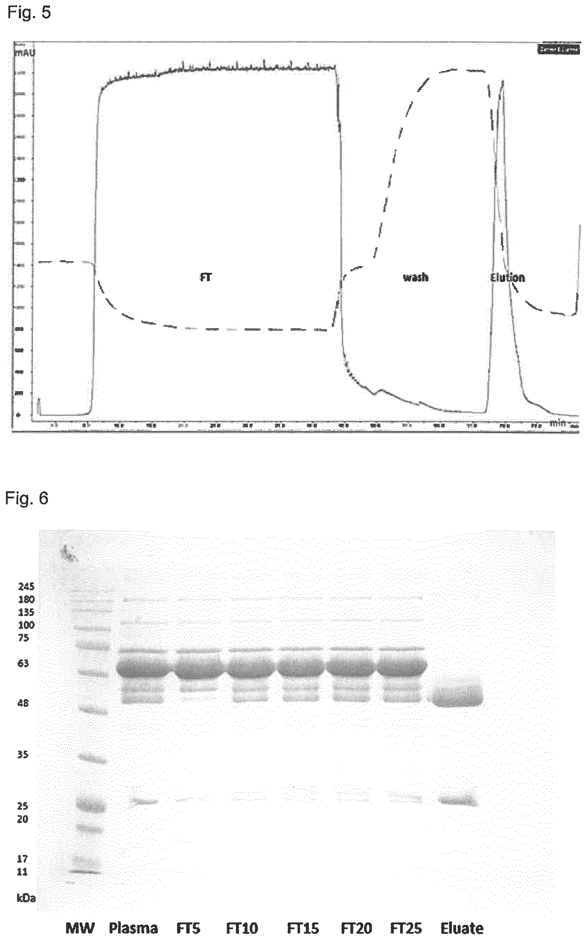

Fresh frozen plasma was thawed, centrifuged and the lipid layer removed. 150 ml of the sample were pumped through an 8 mL CIMmultus Protein G column having a pore size of 1.3 and 6 µm modified with recombinant protein G at a flow rate of 3 mL/min. Equilibration buffer (Buffer A) was 155 mM NaCl in 100 mM, Tris.HCL buffer, pH 7.2, the conductivity was 21.0 mS/cm. After pumping of undiluted human plasma the column was washed with 10 column volumes Washing Buffer containing 0.5 M NaCl in 100 mM Tris.HCL buffer, pH 7.2, the conductivity was 49.0 mS/cm. Bound antibodies (human IgG) were eluted with 0.1 M Glycine-HCl, pH 2.08 and 14.25 mS/cm. The corresponding chromatogram is shown in Figure 5. Clotting factor VIII activity as well as activated partial thromboplastin time were measured in order to demonstrate the stability of plasma during this chromatography. Results are presented in Table 3. SDS-PAGE Analysis was performed in order to confirm the recovery of IgG from protein G column (Figure 6).

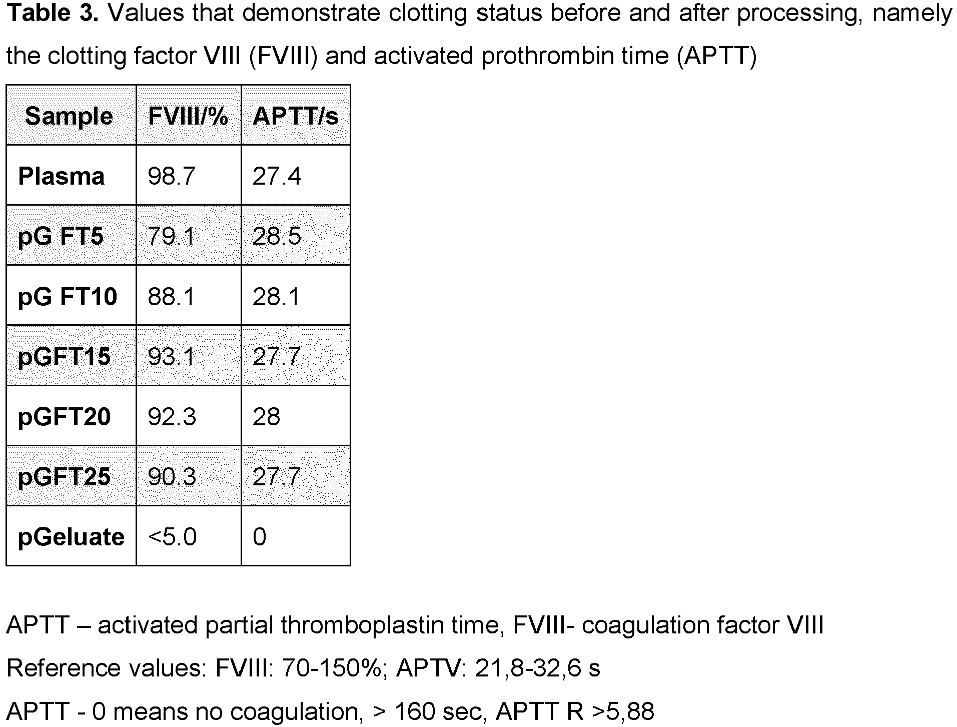

Conclusion: Example 3 demonstrates that the binding of antibodies (also pathological ones) from patient plasma and further analysis for diagnostic purposes is possible without any plugging or precipitation during processing of this biological fluid. The data suggest that the herein described methods are suitable for purification of target molecules such as antibodies that could then be used as drug molecules in different therapeutic applications. Furthermore, the data provides proof that the methods described herein are suitable to remove harmful target substances such as autoimmune antibodies from human plasma that could then be recirculated into the patient's body.

### References

1. EP 2 925 777 B1
2. Sidney, AL, Continuous Downstream Processing for High Value Biological Products, Biotechnol. Bioeng. 2016; 113(3): 465-475.
3. Vogel, J., Nguyen, H., Giovannini, R., Ignowski, J., Garger, S., Salgotra, A. Tom, J., A New Large-scale Manufacturing Platform for Complex Biopharmaceuticals Biotechnol. Bioeng. 2015; 109(12): 3049-3058.
4. M. Šrajer Gajdošik, J. Clifton, Dj. Josic, Sample Displacement Chromatography as a Method for Purification of Proteins and Peptides from Complex Mixtures, J. Chromatogr. A. 2012, 1239, 1-9.
5. Dj. Josič, L. Breen, J. Clifton, M. Šrajer Gajdošik, D. Gašo Sokač, M. Ručevié, E. Müller, Separation of Proteins from Human Plasma in Hydrophobic Interaction Mode, Electrophoresis 2012, 33, 1842-1849.
6. Burnouf, T. Modern Plasma Fractionation, Transf. Med. Reviews, 2007, 21(2) 101-117.
7. Pool, J., Hershgold, E.J., Pappenhagen, A.R., High-potency Antihaemophylic Factor Concentrate prepared from Cryoglobulin Precipitate, Nature 1964, 203, 312.
8. Cohn, E., Strong, L., Hughes, W. Preparation and Properties of Serum and Plasma Proteins.IV. A System for the Separation into Fractions of the Protein and Lipoprotein Components of Biological Tissues and Fluids, J. Am. Chem. Soc. 1946, 68, 459-475.
9. Josic, Dj. Hoffer, L. et al. Manufacturing of a prothrombin complex concentrate aiming at low thrombogenicity, Thromb. Res. 2000, 100(5), 433-441.
10. Janakiraman, V.N., Prasanna, R.P., Kamalanathan, A.S., Vijayalakshmi, M.A. Monolith-based Pseudo-bioaffinity Purification Methods for Factor Vllland Applications Thereof, WO14083510.
11. Rajamanickam V., Hervig, C., Spaduit, O., Monoliths in Bioprocess Technology, Chromatography, 2015, 2(2), 195-212.
12. National Institute of Diabetes and Digestive and Kidney Diseases, Hemodialysis, https://www.niddk.nih.gov/health-information/kidney-disease/kidney-failure/hemodialysis
13. Ciceri, P., Cozzolino, Expandend Haemodialysis as a Current Strategy to Remove Uremic Toxins, Toxins (Basel) 2021, 13(6) 380. doi: 10.3390/toxins13060380
14. Lehmann, H.C., Kieseier, B.C., Hetzel, G.R., Grabensee, B., Hartung, H.-P. Plasmapheresis Therapy in the Neurology, Arzneimitteltherapie 2004, 22, 92-90.
15. Assessment of plasmapheresis. Report of the Therapeutics and Technology Assessment Subcommittee of the American Academy of Neurology. Neurology 1996, 47, 840-843.
16. Franz E, Hörl W. editors. Blutreinigungsverfahren. Stuttgart, New York: Georg Thieme Verlag, 1997.
17. Drew, M.J. "Plasmapheresis in the Dysproteinemias". Therapeutic Apheresis, 2002, 6(1): 45-52.
18. Josič, Dj. Strancar, A., Application of Monoliths and Compact Porous Units for the Separation of Biopolymers", Industrial and Engineering Chemistry, 1999, 38(2), 333-342.
19. Švec, F., Jelinkova, M., Votavova, E., Macroporous Membranes, I, Reactive Macroporous Membranes Based on Glycidyl Methacrylate-Ethylene Dimethacrylate Copolymer for High-Performance Membrane Chromatography, Angew. Macromol. Chem. 1991, 188, 167-176.
20. Strancar, A., Koselj, P., Schwinn, H., Josič, Dj. Application of Compact, Porous Disks for Fast Separations of Biopolymers and In-Process Control in Biotechnology, Anal. Chem. 1996, 68(19), 3483-3488.
21. Sartorius/BIASeparations, Calculating Linear Flow Rate for CIM monoliths, https://www.biaseparations.com/en/technology/calculating-linear-flow-rate-for-cimr-monoliths
22. Potgieter, J.J. Damgaard, M., Hillarp, A. One-stage vs. Chromogenic Assays in Haemophilia A, Eur. J. Haematol. 2015, 94(S77) 38-44.
23.1-stage APTT-based Factor Assays https://practical-haemostasis.com/Factor%20Assays/1-stage_aptt_factor_assay.html
24. Horstl, J. Measurement of Prothrombin Time in EDTA Plasma with Combined Thromboplastin Reagent, Cli. Chem. 2000, 46(11) 1844-1846.
25. https://www.testing.com/tests/complement/

## Claims

1. A method of purifying a target component from a full plasma sample comprising the steps of:
- Applying full plasma to a continuous chromatographic material, the continuous chromatographic material being a compact monolithic material or a membrane adsorber and comprising continuous channels having a diameter of 4 to 15 µm, the chromatographic material comprising a chromatographic selection element for the target component;
- Performing chromatography at a temperature of 30 to 39 °C; and
- Recovering the target component from the continuous chromatographic material,
wherein full plasma is plasma that has not been subjected to dilution and/or cryoprecipitation.

2. A method of removing one or more target components from a full plasma sample comprising the steps of:
- Applying full plasma to a continuous chromatographic material, the continuous chromatographic material being a compact monolithic material or a membrane adsorber and comprising continuous channels with a diameter of 4 to 15 µm, the chromatographic material comprising a chromatographic selection element for the one or more target components;
- Performing chromatography at a temperature of 30 to 39 °C; and
- Recovering the plasma after removal of the one or more target components,
wherein full plasma is plasma that has not been subjected to dilution and/or cryoprecipitation.

3. The method of claim 1 or 2 wherein the full plasma is human blood plasma.

4. The method according to any one of claims 1 to 3, wherein the full plasma is selected from Fresh Frozen Plasma or patient plasma.

5. The method according to any one of claims 1 to 4, wherein the one or more target components are selected from the group consisting of proteins, allergens, nucleic acids, viruses, toxins and nanoparticles.

6. The method according to claim 5, wherein the nanoparticles are exosomes.

7. The method according to claim 5, wherein the protein is selected from the group consisting of clotting factors, clotting inhibitors, antibodies and other physiologically active proteins.

8. The method according to claim 7, wherein the protein is the clotting factor VIII.

9. The method according to claim any one of claims 1 to 5, wherein the one or more target components include one or more harmful components selected from the group consisting of pathological antibodies, macromolecular toxins, nucleic acids, and nanoparticles.

10. The method according to any one of claims 1 to 9, wherein the chromatographic selection element is selected from the group consisting of anion-exchange ligands, cation exchange ligands, affinity ligands, HIC ligands, oligonucleotides, aptamers, and combinations thereof.

11. The method according to any one of claims 1 to 10, wherein the continuous chromatographic material is a continuous methacrylate chromatographic material.

12. The method according to any one of claims 1 to 11, wherein the channels have a diameter in the range of 4 to 10 µm, preferably 5 to 7 µm.

13. The method according to any one of claims 1 to 12, wherein the chromatography is performed at a temperature of 35 to 37 °C.

## Patentansprüche

1. Verfahren zur Reinigung einer Zielkomponente aus einer Vollplasmaprobe, das die folgenden Schritte umfasst:
- Aufbringen von Vollplasma auf ein ununterbrochenes chromatographisches Material, wobei das ununterbrochene chromatographische Material ein kompaktes monolithisches Material oder ein Membranadsorber ist und ununterbrochene Kanäle umfasst, die einen Durchmesser von 4 bis 15 µm aufweisen, wobei das chromatographische Material ein chromatographisches Auswahlelement für die Zielkomponente umfasst,
- Durchführen von Chromatographie bei einer Temperatur von 30 bis 39°C; und
- Rückgewinnen der Zielkomponente von dem ununterbrochenen chromatographischen Material,
wobei das Vollplasma Plasma ist, das nicht Verdünnung und/oder Kryopräzipitation unterzogen wurde.

2. Verfahren zur Entfernung einer oder mehrerer Zielkomponenten von einer Vollplasmaprobe, das die folgenden Schritte umfasst:
- Aufbringen von Vollplasma auf ein ununterbrochenes chromatographisches Material, wobei das ununterbrochene chromatographische Material ein kompaktes monolithisches Material oder ein Membranadsorber ist und ununterbrochene Kanäle mit einem Durchmesser von 4 bis 15 µm umfasst, wobei das chromatographische Material ein chromatographisches Auswahlelement für die eine oder mehreren Zielkomponenten umfasst;
- Durchführen von Chromatographie bei einer Temperatur von 30 bis 39°C; und
- Rückgewinnen des Plasmas nach der Entfernung der einen oder mehreren Zielkomponenten,
wobei Vollplasma Plasma ist, das nicht Verdünnung und/oder Kryopräzipitation unterzogen wurde.

3. Verfahren nach Anspruch 1 oder 2, wobei das Vollplasma Human-Blutplasma ist.

4. Verfahren nach einem der Ansprüche 1 bis 3, wobei das Vollplasma ausgewählt wird aus gefrorenem Frischplasma oder Patientenplasma.

5. Verfahren nach einem der Ansprüche 1 bis 4, wobei die eine oder die mehreren Zielkomponenten ausgewählt werden aus der Gruppe, die besteht aus Proteinen, Allergenen, Nukleinsäuren, Viren, Toxinen und Nanopartikeln.

6. Verfahren nach Anspruch 5, wobei die Nanopartikel Exosome sind.

7. Verfahren nach Anspruch 5, wobei das Protein ausgewählt wird aus der Gruppe, die besteht aus Gerinnungsfaktoren, Gerinnungshemmern, Antikörpern und anderen physiologisch aktiven Proteinen.

8. Verfahren nach Anspruch 7, wobei das Protein der Gerinnungsfaktor VIII ist.

9. Verfahren nach einem der Ansprüche 1 bis 5, wobei die eine oder mehreren Zielkomponenten eine oder mehrere schädliche Komponenten umfassen, die ausgewählt werden aus der Gruppe, die besteht aus pathologischen Antikörpern, makromolekularen Toxinen, Nukleinsäuren und Nanopartikeln.

10. Verfahren nach einem der Ansprüche 1 bis 9, wobei das chromatographische Auswahlelement ausgewählt wird aus der Gruppe, die besteht aus Anionenaustausch-Liganden, Kationenaustausch-Liganden, Affinitätsliganden, HIC-Liganden, Oligonukleotiden, Aptameren und Kombinationen davon.

11. Verfahren nach einem der Ansprüche 1 bis 10, wobei das ununterbrochene chromatographische Material ein ununterbrochenes chromatographisches Methacrylat-Material ist.

12. Verfahren nach einem der Ansprüche 1 bis 11, wobei die Kanäle einen Durchmesser im Bereich von 4 bis 10 µm, vorzugsweise 5 bis 7 µm, aufweisen.

13. Verfahren nach einem der Ansprüche 1 bis 12, wobei die Chromatographie bei einer Temperatur von 35 bis 37°C durchgeführt wird.

## Revendications

1. Procédé de purification d'un composant cible à partir d'un échantillon de plasma entier, comprenant les étapes de :
- application de plasma entier à un matériau chromatographique continu, le matériau chromatographique continu étant un matériau monolithique compact ou un adsorbeur à membrane et comprenant des canaux continus ayant un diamètre de 4 à 15 µm, le matériau chromatographique comprenant un élément de sélection chromatographique pour le composant cible ;
- mise en œuvre d'une chromatographie à une température de 30 à 39°C ; et
- récupération du composant cible à partir du matériau chromatographique continu,
dans lequel le plasma entier est un plasma qui n'a pas été soumis à une dilution et/ou une cryoprécipitation.

2. Procédé d'élimination d'un ou plusieurs composants cibles dans un échantillon de plasma entier, comprenant les étapes de :
- application de plasma entier à un matériau chromatographique continu, le matériau chromatographique continu étant un matériau monolithique compact ou un adsorbeur à membrane et comprenant des canaux continus ayant un diamètre de 4 à 15 µm, le matériau chromatographique comprenant un élément de sélection chromatographique pour le ou les composants cibles ;
- mise en œuvre d'une chromatographie à une température de 30 à 39°C ; et
- récupération du plasma après élimination du ou des composants cibles,
dans lequel le plasma entier est un plasma qui n'a pas été soumis à une dilution et/ou une cryoprécipitation.

3. Procédé selon la revendication 1 ou 2, dans lequel le plasma entier est un plasma sanguin humain.

4. Procédé selon l'une quelconque des revendications 1 à 3, dans lequel le plasma entier est choisi parmi le plasma congelé frais et le plasma d'un patient.

5. Procédé selon l'une quelconque des revendications 1 à 4, dans lequel le ou les composants cibles sont choisis dans le groupe constitué par les protéines, les allergènes, les acides nucléiques, les virus, les toxines, et les nanoparticules.

6. Procédé selon la revendication 5, dans lequel les nanoparticules sont des exosomes.

7. Procédé selon la revendication 5, dans lequel la protéine est choisie dans le groupe constitué par les facteurs de coagulation, les inhibiteurs de coagulation, les anticorps, et d'autres protéines physiologiquement actives.

8. Procédé selon la revendication 7, dans lequel la protéine est le facteur de coagulation VIII.

9. Procédé selon l'une quelconque des revendications 1 à 5, dans lequel le ou les composants cibles comprennent un ou plusieurs composants nocifs choisis dans le groupe constitué par les anticorps pathologiques, les toxines macromoléculaires, les acides nucléiques, et les nanoparticules.

10. Procédé selon l'une quelconque des revendications 1 à 9, dans lequel l'élément de sélection chromatographique est choisi dans le groupe constitué par les ligands d'échange d'anions, les ligands d'échange de cations, les ligands d'affinité, les ligands HIC, les oligonucléotides, les aptamères, et leurs combinaisons.

11. Procédé selon l'une quelconque des revendications 1 à 10, dans lequel le matériau chromatographique continu est un matériau chromatographique de type méthacrylate continu.

12. Procédé selon l'une quelconque des revendications 1 à 11, dans lequel les canaux ont un diamètre situé dans la plage allant de 4 à 10 µm, de préférence de 5 à 7 µm.

13. Procédé selon l'une quelconque des revendications 1 à 12, dans lequel la chromatographie est mise en œuvre à une température de 35 à 37°C.
